# EUROPEAN PATENT APPLICATION

(11) **EP 1 004 576 A1**
(43) Date of publication of application: **31.05.2000**
(21) Application number: 98203946.3
(22) Date of filing: 23.11.1998
(51) Int. Cl.: C07C 409/26, C07C 407/00

(54) **Process for producing peracetic acid**

(71) Applicant: SOLVAY (Société Anonyme), 1050 Bruxelles (BE)
(72) Inventor: Bloomfield, Stephen, 3080 Tervuren (BE); Druitte, Muriel, 1150 Bruxelles (BE); Vandenberg, Dominique, 1332 Rixensart (BE)
(74) Representative: Vande Gucht, Anne

(57) **Abstract**

Process for producing peracetic acid by reacting hydrogen peroxide and acetic acid in an aqueous reaction medium in the presence of an acid catalyst and continuously distilling off the peracetic acid, the molar ratio of hydrogen peroxide to acetic acid in the reaction medium being from about 0.6:1 to about 4:1 and the reaction medium being circulated through a thermosiphon reboiler by natural convective boiling.

## Description

The present invention relates to a process for producing peracetic acid by reacting hydrogen peroxide and acetic acid in an aqueous reaction medium in the presence of an acid catalyst and continuously distilling of the peracetic acid in a fractionating column.

Peracetic acid is a strong oxidizing, epoxidizing and hydroxylating compound which can be used as a disinfectant in aqueous solutions, in cleaning compositions or for the bleaching of various materials. As a chlorine-free compound, it is particularly suited for use in the delignifying and bleaching of paper pulp.

While processes for the production of peracetic acid are known, the safety of such processes, particularly those needed for industrial purposes, remain problematic and open to improvements.

US 3 264 346 discloses a process for producing an aqueous solution of peracetic acid by distilling of the solution from a reaction medium containing acetic acid, hydrogen peroxide, sulphuric acid, water and peracetic acid. Unfortunately, the use of that process calls for a molar ratio of hydrogen peroxide to acetic acid of from 5:1 to 15:1. Such concentrations lead to a large build-up in the amount of hydrogen peroxide in the medium which can pose safety concerns.

EP 0 789 016 discloses a method for producing peracetic acid by reacting hydrogen peroxide and acetic acid in an aqueous medium that is continuously supplied with more than 0.2 kW/kg of thermal energy while the peracetic acid produced is continuously separated and removed by distillation. The reaction medium circulates in the heating device with the aid of a pump which increases the pressure of the reaction medium to allow temperature rise without boiling in the circulation loop. This increase in temperature of the reaction medium can increase the rate of decomposition. The molar ratio of hydrogen peroxide to acetic acid in the reaction medium (reactant molar ratio) is from 4:1 to 30:1 which, in considering the limiting concentration of hydrogen peroxide for condensed phase explosion hazard, implies a relatively low concentration of acetic acid and consequently a relatively low rate of chemical reaction. This, in turn, requires a relatively high volume of reacting liquid and a relatively high magnitude of explosion hazard.

It has now surprisingly been found that when working with smaller quantities of hydrogen peroxide, not only the safety concerns are reduced, but also peracetic acid in a solution having low concentrations of acetic acid and being substantially free from hydrogen peroxide is efficiently obtained.

So, it is an object of the present invention to solve the above-cited problems by providing a new and efficient process for producing peracetic acid which operates at a reactant molar ratio between 0.6:1 and 4:1 whereby the volume of the reacting liquid is minimized.

It is also an object of the present invention to provide a process for producing peracetic acid which does not use a recirculation pump whereby the temperature rise of the reaction medium is minimized and consequently decomposition losses are reduced.

It is a further object of the present invention to provide a process for producing peracetic acid in a solution having low concentrations of acetic acid and being substantially free from hydrogen peroxide.

According to the invention, the production, from hydrogen peroxide and acetic acid, of peracetic acid in a solution having low concentrations of acetic acid and being substantially free from hydrogen peroxide can be safely and efficiently achieved if the reaction medium has a low molar ratio of hydrogen peroxide to acetic acid and if the reaction medium circulates without the use of a recirculation pump.

Thus, the present invention concerns a process for producing peracetic acid by reacting hydrogen peroxide and acetic acid in an aqueous reaction medium in the presence of an acid catalyst and continuously distilling off the peracetic acid. This process is characterized by the molar ratio of hydrogen peroxide to acetic acid in the reaction medium being from about 0.6:1 to about 4:1. This process is also characterized by the reaction medium being circulated through a thermosiphon reboiler by natural convective boiling.

Preferably, the molar ratio of hydrogen peroxide to acetic acid in the reaction medium is from about 1:1 to about 2:1.

The reaction takes place in the bottom of a distillation column hereafter referred as "the still".

It is also preferred that the pressure at which the hydrogen peroxide and acetic acid are reacted (the reaction pressure) and at which the distillation occurs (the distillation pressure) be at least about 50 mbar. More preferably, this pressure is at least 75 mbar. Still more preferably, this pressure is at least 115 mbar. Most advantageously, this pressure is at least 125 mbar. It is at least 150 mbar.

It is preferred that the reaction pressure and the distillation pressure be no greater than about 150 mbar. More preferably, this pressure is no greater than 125 mbar. Still more preferably, this pressure is no greater than 115 mbar. Most advantageously, this pressure is no greater than 75 mbar. It is no greater than 50 mbar.

The reaction pressure and the distillation pressure is at most preferably about 115 mbar.

The temperature at which the hydrogen peroxide and acetic acid are reacted (the reaction temperature) and at which the distillation occurs (the distillation temperature) is preferably at least about 45°C. This temperature is more preferably at least 50°C. This temperature is still more preferably at least 55°C. It is at least 65°C.

To avoid excessive losses of hydrogen peroxide by decomposition, the reaction temperature and the distillation temperature is preferably no greater than about 65°C. This temperature is more preferably no greater than 55°C. This temperature is advantageously no greater than 50°C. It is no greater than 45°C.

At most preferably, the reaction temperature and the distillation temperature is about 55 °C.

The residence time of the reaction medium in the still must be sufficient to approach equilibrium. Preferably, the residence time of the reaction medium in the still is less than about 1 hour.

The premix vessel preferably contains at least 15 % by weight of hydrogen peroxide; at least 25 % by weight of acetic acid and at least 45 % by weight of water. The premix vessel more preferably contains at least 17 % by weight of hydrogen peroxide; at least 31 % by weight of acetic acid and at least 52 % by weight of water. It preferably contains at least 20 % by weight of hydrogen peroxide; at least 35 % by weight of acetic acid and at least 60 % by weight of water.

The premix vessel preferably contains no greater than 20 % by weight of hydrogen peroxide; no greater than 35 % by weight of acetic acid and no greater than 60 % by weight of water. The premix vessel more preferably contains no greater than 17 % by weight of hydrogen peroxide; no greater than 31 % by weight of acetic acid and no greater than 52 % by weight of water. It preferably contains no greater than 15 % by weight of hydrogen peroxide; no greater than 25 % by weight of acetic acid and no greater than 45 % by weight of water.

The premix vessel at most preferably contains about 17 % by weight of hydrogen peroxide; about 31 % by weight of acetic acid and about 52 % by weight of water.

The reaction medium in the still preferably contains at least 5 % by weight of peracetic acid; at least 6 % by weight of hydrogen peroxide; at least 6 % by weight of acetic acid and at least 45 % by weight of water. The reaction medium in the still more preferably contains at least 7 % by weight of peracetic acid; at least 10 % by weight of hydrogen peroxide; at least 10 % by weight of acetic acid and at least 48 % by weight of water. It preferably contains at least 9 % by weight of peracetic acid; at least 15 % by weight of hydrogen peroxide; at least 15 % by weight of acetic acid and at least 50 % by weight of water.

The reaction medium in the still preferably contains no greater than 9 % by weight of peracetic acid; no greater than 15 % by weight of hydrogen peroxide; no greater than 15 % by weight of acetic acid and no greater than 50 % by weight of water. The reaction medium in the still more preferably contains no greater than 7 % by weight of peracetic acid; no greater than 10 % by weight of hydrogen peroxide; no greater than 10 % by weight of acetic acid and no greater than 48 % by weight of water. It preferably contains no greater than 5 % by weight of peracetic acid; no greater than 6 % by weight of hydrogen peroxide; no greater than 6 % by weight of acetic acid and no greater than 45 % by weight of water.

The reaction medium in the still at most preferably contains about 7 % by weight of peracetic acid; about 10 % by weight of hydrogen peroxide; about 10 % by weight of acetic acid and about 48 % by weight of water.

Preferably, the reaction medium in the still also contains an acid catalyst, preferably one or more mineral acids. Examples of mineral acids are sulphuric acid and phosphoric acid. Sulphuric acid is preferred.

The amount of acid catalyst in the reaction medium is preferably at least 5 % by weight. The amount of acid catalyst in the reaction medium is more preferably at least 10 % by weight. It is still more preferably at least 15 % by weight. It is at least 20 % by weight.

The amount of acid catalyst in the reaction medium is preferably no greater than 20 % by weight. The amount of acid catalyst in the reaction medium is preferably no greater than 15 % by weight. It is still more preferably no greater than 10 % by weight. It is no greater than 5 % by weight.

The amount of acid catalyst in the reaction medium is at most preferably about 15 % by weight.

In order to reduce the decomposition of peracetic acid and/or hydrogen peroxide, the reaction medium may also contain one or more stabilizers such as phosphonic acid or salts thereof, pyrophosphoric acid or salts thereof and/or dipicolinic acid or derivatives thereof Dipicolinic acid or derivatives thereof and mixtures of dipicolinic acid or derivatives thereof with phosphonic acid or salts thereof are preferred.

When a stabilizer is needed, the stabilizer is preferably supplied in an amount of about 0.1 % to about 0.2 % by weight. For example, a mixture of 0.1 % by weight of diphosphonic acid and of 0.02 % by weight of dipicolinic acid may be used as stabilizer.

The hydrogen peroxide is supplied to the premix vessel in the form of an aqueous solution at a concentration of from about 20 % to about 30 % by weight. Preferably, the hydrogen peroxide is supplied in the form of an aqueous solution at a concentration of about 25 % by weight.

According to the invention, the production, from hydrogen peroxide and acetic acid, of peracetic acid in a solution having low concentrations of acetic acid and being substantially free from hydrogen peroxide can be safely and efficiently achieved if the reaction medium is distilled at a relatively low reflux ratio.

Thus, the present invention also concerns a process for producing peracetic acid by reacting hydrogen peroxide and acetic acid in an aqueous reaction medium in the presence of an acid catalyst and continuously distilling off the peracetic acid, the reaction medium being maintained at a reflux ratio of from about 0.6 to about 10. More preferably the reaction medium is maintained at a reflux ratio of from about 1 to about 3.

By the term "reflux ratio" what is meant is the ratio of the flow rate of condensed product returned to the distillation column on the flow rate of condensed product take off. By the term "condensed product" what is meant is the vapor leaving the distillation column which has been condensed using cooling water as condensing medium.

After the distillation, the product preferably consists of an aqueous solution containing at least 35 % by weight of peracetic acid. It more preferably consists of an aqueous solution containing at least 37 % by weight of peracetic acid. It consists of an aqueous solution containing at least 40 % by weight of peracetic acid. It consists of an aqueous solution containing at least 58 % by weight of peracetic acid.

After the distillation, the product preferably consists of an aqueous solution containing no greater than 58 % by weight of peracetic acid. It more preferably consists of an aqueous solution containing no greater than 40 % by weight of peracetic acid. It consists of an aqueous solution containing no greater than 37 % by weight of peracetic acid. It consists of an aqueous solution containing no greater than 35 % by weight of peracetic acid.

After the distillation, the product at most preferably consists of an aqueous solution containing about 37 % by weight of peracetic acid.

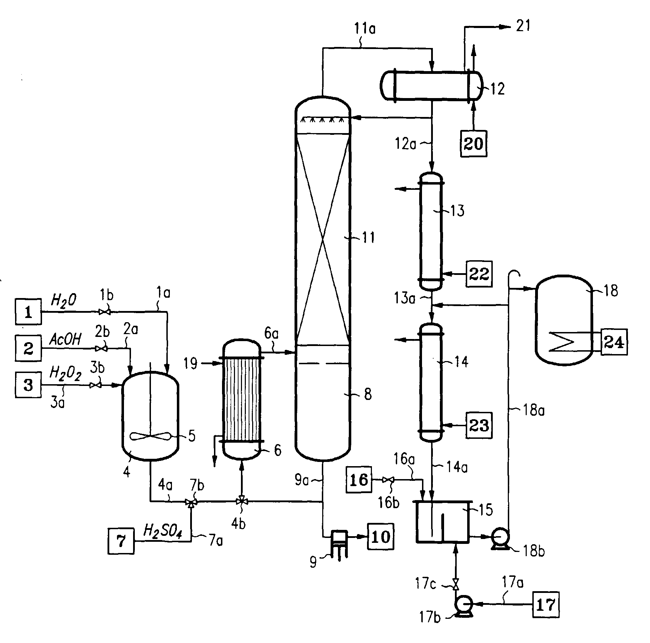

The process of the present invention will now be described in more detail with reference made to the accompanying Figure.

Respective sources for water 1, acetic acid 2 and hydrogen peroxide 3 are provided for successively supplying those ingredients in the concentrations mentioned above, to a premix vessel 4, wherein a premix feed is made therefrom.

Conduits 1a, 2a and 3a are positioned between, and in fluid communication with, the premix vessel 4 and, respectively, the water source 1, the acetic acid source 2 and the hydrogen peroxide source 3. These conduits 1a, 2a and 3a provide for fluid communication between the premix vessel 4 and respective sources 1, 2 and 3. Valves 1b, 2b and 3b which are interposed in, respectively, conduits 1a, 2a and 3a, control the flow rate and quantity of the water, acetic acid and hydrogen peroxide which passes through the conduits 1a, 2a and 3a.

In the premix vessel 4, which is maintained at ambient temperature and pressure, the water, acetic acid and hydrogen peroxide are agitated with a stirrer 5, thereby producing a premix feed.

The premix feed then passes from the premix vessel 4 and into a conduit 4a which is positioned between the premix vessel 4 and the thermosiphon reboiler 6, and which provides for fluid communication therebetween.

While in conduit 4a (and before entering the reboiler 6), a quantity of sulphuric acid is mixed with the premix feed in order to attain the concentrations mentioned above. The sulphuric acid is provided from a sulphuric acid source 7 which is supplied to conduit 4a via a conduit 7a which extends between, and is in fluid communication with, the sulphuric acid source 7 and the conduit 4a. Valve 7b regulates and controls the flow rate and quantity of sulphuric acid which passes through conduit 7a and into conduit 4a, where it mixes with the premix feed, thereby producing a reaction medium which then passes from the conduit 4a into the reboiler 6.

The flow of the reaction medium from conduit 4a and into the reboiler 6 is regulated and controlled by valve 4b (which is interposed in conduit 4a), so that the reaction medium from conduit 4a is supplied to the thermosiphon reboiler 6 in a continuous operation.

In the thermosiphon reboiler 6 (the precise structure and functioning of which is described and defined in "Perry's Chemical Engineers' Handbook, 1984, at p. 11-3, which reference is incorporated herein and made a part hereof), the thermal energy contained in a continuously condensing steam or hot water (as the heating medium 19) is transferred therefrom to the reaction medium, producing a boiling reaction medium.

The heated reaction medium recirculates from the reboiler 6 to the still 8 via conduit 6a which is positioned between, and in fluid communication with, the reboiler 6 and the still 8. In the still 8, as well as in the reboiler 6, the peracetic acid is formed in an in situ reversible reaction that is catalyzed by the sulphuric acid.

It is noted that the reaction medium circulates through the thermosiphon reboiler 6 by natural convective boiling at the same above-mentioned temperature and pressure as is present in the still 8 (reaction temperature and pressure) and in the distillation column 11 (distillation temperature and pressure).

To avoid excessive losses of hydrogen peroxide and peracetic acid due to decomposition, the still 8 is provided with a small purge 9 (<1% v/v of the feed flow) which controls the concentration of nonvolatile impurities from the raw materials that would otherwise build-up in the still 8 and catalyze the decomposition. Purge 9 permits such materials to be purged from the still 8 via conduit 9a which is positioned between, and in fluid communication with, the purge 9 and the effluent treatment means 10.

Sulphuric acid catalyst lost via the purge 9, is continuously replenished from the source 7 to maintain the sulphuric acid in the still 8 at the concentrations mentioned above.

In the still 8, the peracetic acid (which forms a minimum boiling azeotrope with water) is vaporized together with the excess water. This vaporized mixture rises into a distillation column 11, which is in fluid communication with the still 8. The vapor is purified in the column 11. The distillation column 11 operates under reflux and the resultant vapor exits the column 11 and passes into an overhead condenser 12 via a conduit 11a which is positioned between, and in fluid communication with, the column 11 and the condenser 12.

In the condenser 12, the vapor is condensed using a cooling fluid (such as water) (10-35°C) as the condensing medium, thereby producing a condensed product. The condensing medium is supplied to the condenser 12 from a cooling fluid source 20 via a conduit that is positioned between, and in fluid communication with, the condenser 12 and the cooling fluid source 20.

The remaining vapor leaves the condenser 12 together with gases from decomposition and leakage from the atmosphere to a vacuum system 21.

The condensed product contains from about 35 to about 58% by weight of peracetic acid, less than about 5% by weight of acetic acid, preferably less than about 2% by weight of acetic acid and is substantially free from hydrogen peroxide, the remainder being water. Any excess of condensed product is returned to the distillation column 11 as reflux in order to have a reflux ratio of from about 0.6 to about 10.

The condensed product passes from the condenser 12 to a cooler 13 via a conduit 12a which is positioned between, and in fluid communication with, the condenser 12 and the cooler 13. In the cooler 13, the condensed product is cooled using a cooling fluid (such as water) (10-35°C) which is supplied to the cooler 13 from a cooling fluid source 22 via a conduit which is positioned between, and in fluid communication with, the cooler 13 and the cooling fluid source 22.

The cooled product passes from the cooler 13 to a chiller 14 via a conduit 13a which is positioned between, and in fluid communication with, the cooler 13 and the chiller 14. In the chiller 14, the cooled product is chilled using a chilled fluid (such as chilled water) (typically -5°C) supplied from a chilled fluid source 23 via a conduit that is positioned between, and in fluid communication with, the chiller 14 and the chilled fluid source 23. In this fashion, a chilled product is produced.

The chilled product is then drawn-off from the chiller 14 at a constant rate via conduit 14a to a product collection tank 15 in order to provide a constant production rate.

In the product collection tank 15, the product is stabilized with dipicolinic acid and/or one of the above-mentioned phosphonic acids supplied from a stabilizer source 16 via a conduit 16a which is positioned between, and in fluid communication with, the source 16 and the product collection tank 15. The flow of the stabilizer from the source 16 and into the product collection tank 15 via the conduit 16a is regulated and controlled by valve 16b (which is interposed in conduit 16a), so that the stabilizer from source 16 is supplied to the product collection tank 15 in a continuous operation. In this fashion, a stabilized product is formed.

The stabilized product may then be diluted with demineralized water from a source 17 via a conduit 17a which is positioned between, and in fluid communication with, the source 17 and the product collection tank 15. The demineralized water is pumped to the product collection tank 15 by way of a pump 17b which is interposed in conduit 17a. The flow of the demineralized water is regulated and controlled by valve 17c (which is interposed in conduit 17a between the pump 17b and the product collection tank 15). In this fashion, a dilute, stabilized product is formed.

The dilute, stabilized product may be stored in the product collection tank 15 if it is used directly after its production.

Otherwise, the dilute, stabilized product may be pumped to a storage tank 18 via a conduit 18a which is positioned between, and in fluid communication with, the storage tank 18 and the product collection tank 15. Such pumping is performed by pump 18b which is interposed in conduit 18a.

In the storage tank 18, the dilute, stabilized product is stored at ambient pressure and is maintained at a temperature of about 0°C to about 5°C using a chilled fluid (such as chilled water) supplied thereto from a chilled fluid source 24. In this fashion, loss of peracetic acid by reverse reaction is minimized.

The choice of materials of construction for handling and storage of systems containing peracetic acid is dependent upon concentration, temperature, storage period and the operating environment of its intended application. In accordance with the above-discussed process of the present invention, the process equipment, such as reactant vessels, premix vessel, pipework, reboiler, distillation column, condenser, product vessel, etc. are preferably made of a corrosion-proof material such as glass, aluminum or stainless steel. Other suitable materials are tantalum, tantalum coated stainless steel, glass lined carbon steel, fluoropolymers, such as PTFE, PVDF, PFA or FEP or fluoropolymer-coated materials. The preferred materials are glass lined carbon steel for the distillation column, glass for the condenser, tantalum for the reboiler and stainless steel for the other equipment.

The process according to the present invention shows numerous advantages. The process is safe. It produces peracetic acid which contains less than about 2 % by weight of acetic acid and which is substantially free from hydrogen peroxide. It shows a low degree of reversion and decomposition of the peracetic acid. Moreover, it produces a minimal effluent stream.

The present invention will be further illustrated by the following example which is not meant to be restrictive.

### Example

In a plant according to the enclosed Figure, 52 % by weight of water from a source of water 1, 31 % by weight of glacial acetic acid from a source of glacial acetic acid 2 and 17 % by weight of hydrogen peroxide from a source of hydrogen peroxide 3 (in the form of a 25 % aqueous solution) were premixed in the premix vessel 4. These reactants were supplied continuously to the premix vessel 4 at flow rates of 5.5 kg/hr for water, 3.4 kg/hr for acetic acid and 1.8 kg/hr for hydrogen peroxide. This premixed feed was introduced into the still 8, via the thermosiphon reboiler 6, together with 20 % by weight of sulphuric acid, supplied continuously from a source of sulphuric acid 7 at a flow rate of 0.02 kg/hr. The reaction pressure in the still 8 was 115 mbar and the temperature was 55°C. Boiling of the mixture was provided via the thermosiphon reboiler 6 with steam at about 2 bar. The residence time in the still was about 0.4 hour. The fractionation was achieved concurrently in the distillation column 11 using a glass lined carbon steel packed column having 6 to 8 theoretical plates and operating at a reflux ratio of about 1.5. A small purge 9 from the still to the effluent treatment means 10 of approximately 1 % of the feed flow was maintained. After being condensed in the condenser 12, a product was obtained containing 37 % by weight of peracetic acid, less than 2 % by weight of acetic acid, less than 0.1 % by weight of hydrogen peroxide and about 61 % by weight of water. The production rate was about 800 g peracetic acid per kg reaction medium and hour. After being cooled with cooling water from a cooling fluid source 22 (25°C) in the cooler 13 and being chilled with chilled water from a chilled fluid source 23 (-5°C) in the chiller 14, the product was stabilized, in the product collection tank 15, with dipicolinic acid supplied from a source of dipicolinic acid 16 and then diluted to 25 % by weight with demineralized water and stored in the product vessel 18 at a temperature between 0 and 5°C.

The purge 9 to the effluent treatment means 10 was a single aqueous stream of flow rate 0.1 kg/hr and of the following typical composition: 7 % by weight of peracetic acid, 16 % by weight of hydrogen peroxide, 16 % by weight of acetic acid, 20 % by weight of sulphuric acid and 41 % by weight of water.

Further variations and modifications of the present invention will become apparent to those skilled in the art and are intended to be encompassed by the appended claims.

## Claims

1. Process for producing peracetic acid by reacting hydrogen peroxide and acetic acid in an aqueous reaction medium in the presence of an acid catalyst and continuously distilling off the peracetic acid, characterized in that the molar ratio of hydrogen peroxide to acetic acid in the reaction medium is from about 0.6:1 to about 4:1.

2. Process according to claim 1, characterized in that the molar ratio of hydrogen peroxide to acetic acid in the reaction medium is from about 1:1 to about 2:1.

3. Process according to claim 1 or 2, characterized in that the reaction medium circulates through a thermosiphon reboiler by natural convective boiling

4. Process according to claim 1 to 3, characterized in that the reaction pressure and the distillation pressure is from about 50 to about 150 mbar.

5. Process according to claim 1 to 4, characterized in that the reaction temperature and the distillation temperature is from about 45 to about 65°C.

6. Process according to claim 1 to 5, characterized in that the reaction medium is maintained at a reflux ratio of from about 0.6 to about 10.

7. Process according to claim 1 to 6, characterized in that the reaction medium is maintained at a reflux ratio of from about 1 to about 3.

8. Process according to claim 1 to 7, characterized in that after the distillation, the product consists of an aqueous solution containing from about 35 to about 58 % by weight of peracetic acid.

9. Process according to claim 1 to 8, characterized in that the reaction medium circulates through the thermosiphon reboiler by natural convective boiling at the same pressure and at the same temperature as in the still and in the distillation column.

10. Process according to claim 1 to 9, characterized in that the condensed product contains less than about 2 % by weight of acetic acid.
